# EUROPEAN PATENT APPLICATION

(11) **EP 4 498 082 A1**
(43) Date of publication of application: **29.01.2025**
(21) Application number: 24184958.7
(22) Date of filing: 27.06.2024
(51) Int. Cl.: G01N 33/00, H04W 4/38

(54) **POSITIONING POLLUTANT-MEASURING SYSTEM**

(30) Priority: 27.07.2023 US 202363515986 P; 26.09.2023 US 202363585286 P
(71) Applicant: Far Eastone Telecommunications Co., Ltd., Taipei City 10602 (TW)
(72) Inventor: YANG, Chia-Jui, 10602 Taipei City, Taiwan (TW); RAO, Herman Chunghwa, 10602 Taipei City, Taiwan (TW); KUO, Chun-Chieh, 10602 Taipei City, Taiwan (TW); CHIANG, Hua-Pei, 10602 Taipei City, Taiwan (TW); HSIAO, Shui-Shu, 10602 Taipei City, Taiwan (TW); CHANG, Zheng-Xiang, 10602 Taipei City, Taiwan (TW); JANG, Chyi-Dar, 10602 Taipei City, Taiwan (TW); WANG, Tsung-Jen, 10602 Taipei City, Taiwan (TW); LIAO, Che-Yu, 10602 Taipei City, Taiwan (TW); CHAN, Chih-Min, 10602 Taipei City, Taiwan (TW); YANG, Teng-Chieh, 10602 Taipei City, Taiwan (TW); HSU, Chang-Hung, 10602 Taipei City, Taiwan (TW)
(74) Representative: Balder IP Law, S.L.

(57) **Abstract**

A positioning pollutant-measuring system (1) includes a cloud server (10), a wireless base station (11), an automatic moving vehicle (12), and a positioning pollutant-measuring device (13). The automatic moving vehicle (12) carries the positioning pollutant-measuring device (13) and passes through different locations. The positioning pollutant-measuring device (13) receives location related parameters from the wireless base station (11) and measures the air flow rates or the air humidity of the different locations to adjust a resolution for measuring pollutants corresponding to the different locations. The positioning pollutant-measuring device (13) measures the concentrations of pollutants corresponding to the different locations at different time points based on the resolution, transmits the concentrations, the different time points, and the location related parameters to the cloud server (10) through the wireless base station (11), so as to calculate the three-dimensional coordinates of the different locations, and combines the three-dimensional coordinates with the corresponding time points and concentrations.

## Description

### BACKGROUND OF THE INVENTION

This application claims priority for the US provisional patent application Nos. 63/515,986 filed on 27 July 2023, and 63/585,286 filed on 26 September 2023, the contents of which are incorporated by reference in their entirety.

### Field of the Invention

The present invention relates to a pollutant-measuring system, particularly to a positioning pollutant-measuring system.

### Description of the Related Art

With the advancement of industry and commerce, modern people spend more and more time indoors. Most of the buildings in recent years have closed designs. Often due to poor ventilation in the indoor environment, air pollutants are easy to accumulate in the indoor environment, which affects human health. Compared with the outdoor environment, the concentration of indoor air pollutants is often higher than that of outdoor air pollutants. Therefore, the importance and safety of indoor air quality are more concerned and valued by people.

Generally, there are many sources of pollutants that cause indoor air pollution, such as cooking, smoking, spraying, or human activities of using indoor building materials, plywood, and resin adhesives, which may produce hazardous pollutants such as volatile organic compounds and suspended particles. Therefore, in addition to increasing the ventilation rate of the indoor environment, air purifiers can also be used to effectively remove indoor air pollutants in order to improve indoor air quality. However, air purifiers can only remove parts of the pollutants in the indoor space. No one knows which part of the indoor space has more pollutants and which part of the indoor space has fewer pollutants.

To overcome the abovementioned problems, the present invention provides a positioning pollutant-measuring system, so as to solve the afore-mentioned problems of the prior art.

### SUMMARY OF THE INVENTION

The present invention provides a positioning pollutant-measuring system, which implements environmental detection distribution in three-dimensional space.

In an embodiment of the present invention, a positioning pollutant-measuring system includes a cloud server, at least one wireless base station, an automatic moving vehicle, and a positioning pollutant-measuring device. The wireless base station is electrically connected to the cloud server. The automatic moving vehicle is configured to move in a physical environment and pass through different locations in the physical environment. The positioning pollutant-measuring device is wirelessly connected to the wireless base station and arranged on the automatic moving vehicle. The positioning pollutant-measuring device is configured to obtain from the wireless base station reference signal received power (RSRP), signal-to-interference-plus-noise ratios (SINRs), packet round-trip time (RTT), signal angles of arrival, signal emission angles of departure, and the location of the wireless base station corresponding to the different locations. The positioning pollutant-measuring device is configured to measure the air flow rates or the air humidity of the different locations and adjust a resolution for measuring pollutants corresponding to the different locations based on the air flow rates or the air humidity. The positioning pollutant-measuring device is configured to measure the concentrations of the pollutants corresponding to the different locations at different time points based on the resolution and transmit the concentrations, the different time points, the RSRP, the SINRs, the packet RTT, the signal angles of arrival, the signal emission angles of departure, and the location of the wireless base station to the cloud server through the wireless base station. The cloud server is configured to calculate the three-dimensional coordinates of the different locations based on the RSRP, the SINRs, the packet RTT, the signal angles of arrival, the signal emission angles of departure, and the location of the wireless base station and combine the three-dimensional coordinates with the corresponding time points and the corresponding concentrations.

In an embodiment of the present invention, the positioning pollutant-measuring device includes a wireless communication module, a wireless parameter extractor, a pollutant sensor, an air flow meter, and a processor. The wireless communication module is wirelessly connected to the at least one wireless base station. The wireless parameter extractor is electrically connected to the wireless communication module and configured to obtain the RSRP, the SINRs, the packet RTT, the signal angles of arrival, the signal emission angles of departure, and the location of the wireless base station from the wireless base station through the wireless communication module. The pollutant sensor, having the resolution, is configured to measure the concentrations of the pollutants corresponding to the different locations at the different time points based on the resolution. The air flow meter is configured to measure the air flow rates of the different locations in the physical environment. The processor is electrically connected to the wireless parameter extractor, the wireless communication module, the pollutant sensor, and the air flow meter and configured to control the pollutant sensor to adjust the resolution based on the air flow rates and transmit the concentrations, the different time points, the RSRP, the SINRs, the packet RTT, the signal angles of arrival, the signal emission angles of departure, and the location of the wireless base station to the cloud server through the wireless communication module and the wireless base station.

In an embodiment of the present invention, the pollutant sensor is an optical finger navigation (OFN) sensor.

In an embodiment of the present invention, the positioning pollutant-measuring device further includes an inertial measurement unit (IMU) electrically connected to the processor. The IMU is configured to measure its displacement, direction, or acceleration and transmit the displacement, the direction, or the acceleration to the processor. The processor is configured to transmit the displacement, the direction, or the acceleration to the cloud server through the wireless communication module and the wireless base station. The cloud server is configured to calculate the three-dimensional coordinates based on one of the displacement, the direction, and the acceleration.

In an embodiment of the present invention, the positioning pollutant-measuring device further includes an automatic angle adjusting machine electrically connected to the processor and combined with the pollutant sensor. The processor is configured to control the automatic angle adjusting machine to adjust the sensing angle of the pollutant sensor.

In an embodiment of the present invention, the positioning pollutant-measuring system further includes a two-dimensional (2D) lidar and an automatic height adjusting machine. The 2D lidar is electrically connected to the processor and arranged on the automatic moving vehicle. The two-dimensional lidar is configured to scan an obstacle around itself to obtain a relative distance between the two-dimensional lidar and the obstacle. The automatic height adjusting machine is arranged between the automatic moving vehicle and the positioning pollutant-measuring device and electrically connected to the processor. When the relative distance is less than a given distance, the processor drives the automatic height adjusting machine to increase a height where the positioning pollutant-measuring device is located. When the relative distance is greater than or equal to the given distance, the processor drives the automatic height adjusting machine to decrease a height where the positioning pollutant-measuring device is located.

In an embodiment of the present invention, the positioning pollutant-measuring device includes a wireless communication module, a wireless parameter extractor, a pollutant sensor, an air hygrometer, and a processor. The wireless communication module is wirelessly connected to the at least one wireless base station. The wireless parameter extractor is electrically connected to the wireless communication module and configured to obtain the RSRP, the SINRs, the packet RTT, the signal angles of arrival, the signal emission angles of departure, and the location of the wireless base station from the wireless base station through the wireless communication module. The pollutant sensor, having the resolution, is configured to measure the concentrations of the pollutants corresponding to the different locations at the different time points based on the resolution. The air hygrometer is configured to measure the air humidity of the different locations in the physical environment. The processor is electrically connected to the wireless parameter extractor, the wireless communication module, the pollutant sensor, and the air hygrometer and configured to control the pollutant sensor to adjust the resolution based on the air humidity and transmit the concentrations, the different time points, the RSRP, the SINRs, the packet RTT, the signal angles of arrival, the signal emission angles of departure, and the location of the wireless base station to the cloud server through the wireless communication module and the wireless base station.

In an embodiment of the present invention, the pollutant sensor is an optical finger navigation (OFN) sensor.

In an embodiment of the present invention, the positioning pollutant-measuring device further includes an inertial measurement unit (IMU) electrically connected to the processor. The IMU is configured to measure its displacement, direction, or acceleration and transmit the displacement, the direction, or the acceleration to the processor. The processor is configured to transmit the displacement, the direction, or the acceleration to the cloud server through the wireless communication module and the wireless base station. The cloud server is configured to calculate the three-dimensional coordinates based on one of the displacement, the direction, and the acceleration.

In an embodiment of the present invention, the positioning pollutant-measuring device further includes an automatic angle adjusting machine electrically connected to the processor and combined with the pollutant sensor. The processor is configured to control the automatic angle adjusting machine to adjust the sensing angle of the pollutant sensor.

In an embodiment of the present invention, the positioning pollutant-measuring system further includes a two-dimensional (2D) lidar and an automatic height adjusting machine. The 2D lidar is electrically connected to the processor and arranged on the automatic moving vehicle. The two-dimensional lidar is configured to scan an obstacle around itself to obtain a relative distance between the two-dimensional lidar and the obstacle. The automatic height adjusting machine is arranged between the automatic moving vehicle and the positioning pollutant-measuring device and electrically connected to the processor. When the relative distance is less than a given distance, the processor drives the automatic height adjusting machine to increase a height where the positioning pollutant-measuring device is located. When the relative distance is greater than or equal to the given distance, the processor drives the automatic height adjusting machine to decrease a height where the positioning pollutant-measuring device is located.

In an embodiment of the present invention, the wireless base station is a 3G base station, a 4G base station, 5G base station, a Bluetooth (RTM) base station, or a WiFi base station.

In an embodiment of the present invention, the pollutants include bacteria, mold, viruses, flame, smoke, liquefied gas, carbon monoxide, carbon dioxide, ozone, alkane gases, benzene gases, ketone gases, natural gas, coal gas, gasoline, chlorine, ammonia, flammable gases, harmful volatile matter, or airborne matter.

To sum up, the positioning pollutant-measuring system combines the different locations with the concentrations of the pollutants at different time points to implement environmental detection distribution in three-dimensional space.

Below, the embodiments are described in detail in cooperation with the drawings to make easily understood the technical contents, characteristics and accomplishments of the present invention.

### BRIEF DESCRIPTION OF THE DRAWINGS

- Fig.1: is a schematic diagram illustrating a positioning pollutant-measuring system according to an embodiment of the present invention;
- Fig.2: is a schematic diagram illustrating a positioning pollutant-measuring device according to an embodiment of the present invention;
- Fig.3: is a schematic diagram illustrating a positioning pollutant-measuring device according to anther embodiment of the present invention; and
- Fig.4: is a schematic diagram illustrating a pollutant sensor according to anther embodiment of the present invention.

### DETAILED DESCRIPTION OF THE INVENTION

Reference will now be made in detail to embodiments illustrated in the accompanying drawings. Wherever possible, the same reference numbers are used in the drawings and the description to refer to the same or like parts. In the drawings, the shape and thickness may be exaggerated for clarity and convenience. This description will be directed in particular to elements forming part of, or cooperating more directly with, methods and apparatus in accordance with the present disclosure. It is to be understood that elements not specifically shown or described may take various forms well known to those skilled in the art. Many alternatives and modifications will be apparent to those skilled in the art, once informed by the present disclosure.

When an element is referred to as being "on" another element, it can be directly on the other element or intervening elements may be present therebetween. In contrast, when an element is referred to as being "directly on" another element, there are no intervening elements present. As used herein, the term "and/or" includes any and all combinations of one or more of the associated listed items.

Reference throughout this specification to "one embodiment" or "an embodiment" means that a particular feature, structure, or characteristic described in connection with the embodiment is included in at least one embodiment. Thus, the appearances of the phrases "in one embodiment" or "in an embodiment" in various places throughout this specification are not necessarily all referring to the same embodiment.

The invention is particularly described with the following examples which are only for instance. Those skilled in the art will readily observe that numerous modifications and alterations of the device and method may be made while retaining the teachings of the invention. Accordingly, the following disclosure should be construed as limited only by the metes and bounds of the appended claims. In the whole patent application and the claims, except for clearly described content, the meaning of the articles "a" and "the" includes the meaning of "one or at least one" of the elements or components. Moreover, in the whole patent application and the claims, except that the plurality can be excluded obviously according to the context, the singular articles also contain the description for the plurality of elements or components. In the entire specification and claims, unless the contents clearly specify the meaning of some terms, the meaning of the article "wherein" includes the meaning of the articles "wherein" and "whereon". The meanings of every term used in the present claims and specification refer to a usual meaning known to one skilled in the art unless the meaning is additionally annotated. Some terms used to describe the invention will be discussed to guide practitioners about the invention. The examples in the present specification do not limit the claimed scope of the invention.

Furthermore, it can be understood that the terms "comprising," "including," "having," "containing," and "involving" are open-ended terms, which refer to "may include but is not limited to so." In addition, each of the embodiments or claims of the present invention is not necessary to achieve all the effects and advantages possibly to be generated, and the abstract and title of the present invention is used to assist for patent search and is not used to further limit the claimed scope of the present invention.

Further, in the present specification and claims, the term "comprising" is open type and should not be viewed as the term "consisted of." In addition, the term "electrically coupled" can be referring to either directly connecting or indirectly connecting between elements. Thus, if it is described in the below contents of the present invention that a first device is electrically coupled to a second device, the first device can be directly connected to the second device, or indirectly connected to the second device through other devices or means. Moreover, when the transmissions or generations of electrical signals are mentioned, one skilled in the art should understand some degradations or undesirable transformations could be generated during the operations. If it is not specified in the specification, an electrical signal at the transmitting end should be viewed as substantially the same signal as that at the receiving end. For example, when the end A of an electrical circuit provides an electrical signal S to the end B of the electrical circuit, the voltage of the electrical signal S may drop due to passing through the source and drain of a transistor or due to some parasitic capacitance. However, the transistor is not deliberately used to generate the effect of degrading the signal to achieve some result, that is, the signal S at the end A should be viewed as substantially the same as that at the end B.

Unless otherwise specified, some conditional sentences or words, such as "can", "could", "might", or "may", usually attempt to express what the embodiment in the present invention has, but it can also be interpreted as a feature, element, or step that may not be needed. In other embodiments, these features, elements, or steps may not be required.

In the following description, a positioning pollutant-measuring system will be provided, which combines different locations with the concentrations of pollutants at different time points to implement environmental detection distribution in three-dimensional space.

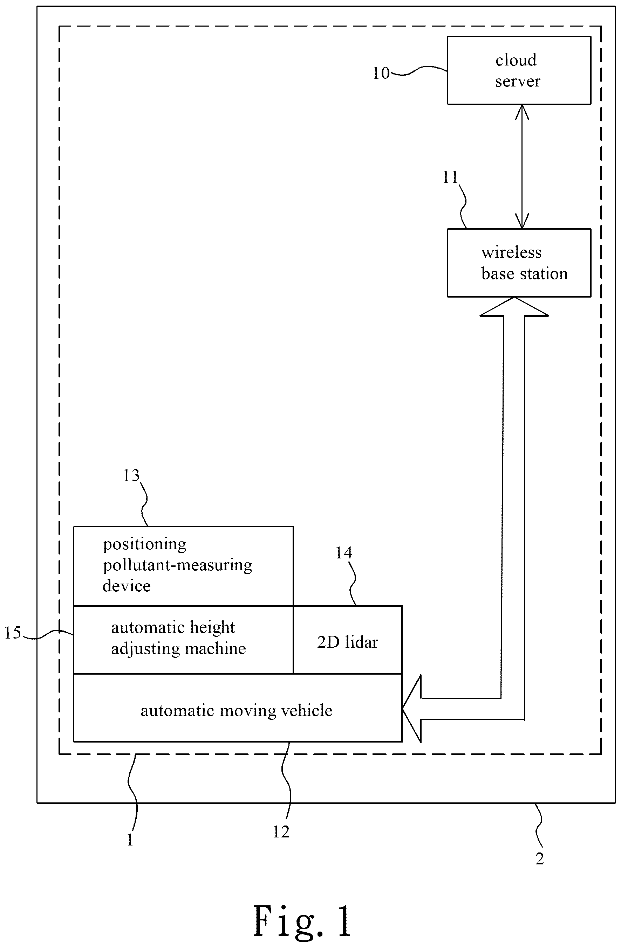

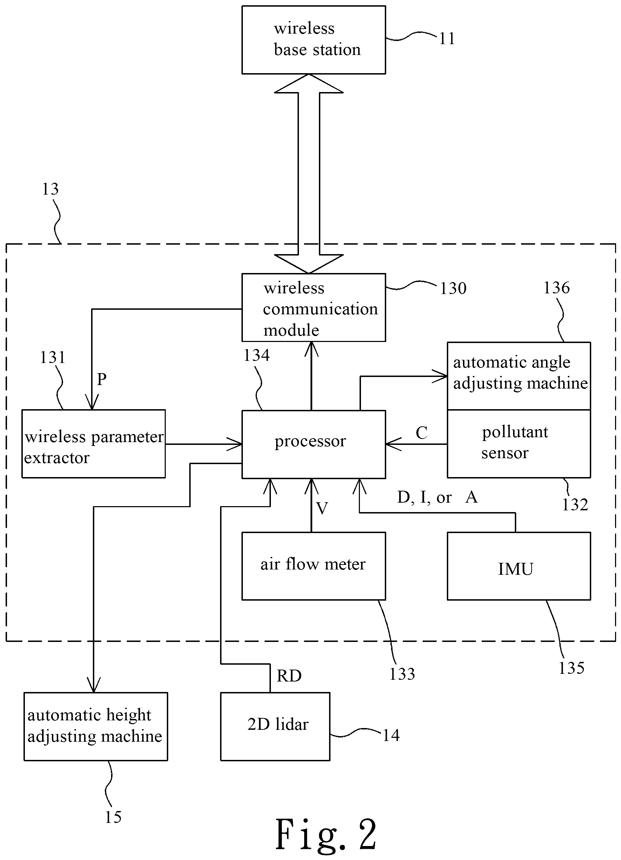

Fig.1 is a schematic diagram illustrating a positioning pollutant-measuring system according to an embodiment of the present invention. Fig.2 is a schematic diagram illustrating a positioning pollutant-measuring device according to an embodiment of the present invention. Referring to Fig.1 and Fig.2, a positioning pollutant-measuring system 1 is introduced as follows. The positioning pollutant-measuring system 1 includes a cloud server 10, at least one wireless base station 11, an automatic moving vehicle 12, and a positioning pollutant-measuring device 13. For convenience and clarity, the number of the wireless base station 11 is one. The wireless base station 11 may be, but not limited to, a 3G base station, a 4G base station, 5G base station, a Bluetooth (RTM) base station, or a WiFi base station. The wireless base station 11 is electrically connected to the cloud server 10. The positioning pollutant-measuring device 13 is wirelessly connected to the wireless base station 11 and arranged on the automatic moving vehicle 12.

The automatic moving vehicle 12 moves in a physical environment 2 and passes through different locations in the physical environment 2. The positioning pollutant-measuring device 13 obtains from the wireless base station 11 location related parameters P corresponding to the different locations. The location related parameters P include reference signal received power (RSRP), signal-to-interference-plus-noise ratios (SINRs), packet round-trip time (RTT), signal angles of arrival, signal emission angles of departure, and the location of the wireless base station 11. In other embodiments, the location related parameters P further include reference signal receiving quality (RSRQ), channel quality indicator (CQI), timing advance (TA), cell identification (ID), base station neighbor list, and channel status information. The wireless base station 11 can generate the location related parameters P with fixed power or variable power. The positioning pollutant-measuring device 13 measures the air flow rates or the air humidity of the different locations and adjusts a resolution for measuring pollutants corresponding to the different locations based on the air flow rates or the air humidity. The pollutants include bacteria, mold, viruses, flame, smoke, liquefied gas, carbon monoxide, carbon dioxide, ozone, alkane gases, benzene gases, ketone gases, natural gas, coal gas, gasoline, chlorine, ammonia, flammable gases, harmful volatile matter, or airborne matter. For example, when the air flow rate or the air humidity is higher, the pollutants are transmitted faster. Thus, the resolution for measuring pollutants corresponding to the air flow rate will be adjusted higher to improve the measurement accuracy.

The positioning pollutant-measuring device 13 measures the concentrations C of the pollutants corresponding to the different locations at different time points based on the resolution and transmits the concentrations C, the different time points, and the location related parameters P including the RSRP, the SINRs, the packet RTT, the signal angles of arrival, the signal emission angles of departure, and the location of the wireless base station 11 to the cloud server 10 through the wireless base station 11. The cloud server 10 calculates the three-dimensional coordinates of the different locations based on the RSRP, the SINRs, the packet RTT, the signal angles of arrival, the signal emission angles of departure, and the location of the wireless base station 11 and combines the three-dimensional coordinates with the corresponding time points and the corresponding concentrations C to implement environmental detection distribution in three-dimensional space. For example, the three-dimensional coordinates PA correspond to a first time point and the concentration a of pollutants, the three-dimensional coordinates PB correspond to a second time point and the concentration b of pollutants, and the three-dimensional coordinates PC correspond to a third time point and the concentration c of pollutants.

In some embodiments of the present invention, the positioning pollutant-measuring device 13 may include a wireless communication module 130, a wireless parameter extractor 131, a pollutant sensor 132, an air flow meter 133, and a processor 134. The wireless communication module 130 is wirelessly connected to the wireless base station 11. The wireless parameter extractor 131 is electrically connected to the wireless communication module 130. The processor 134 is electrically connected to the wireless communication module 130, the wireless parameter extractor 131, the pollutant sensor 132, and the air flow meter 133. The wireless parameter extractor 131 obtains the location related parameters P including the RSRP, the SINRs, the packet RTT, the signal angles of arrival, the signal emission angles of departure, and the location of the wireless base station 11 from the wireless base station 11 through the wireless communication module 130. The pollutant sensor 132 has the resolution for measuring pollutants and measures the concentrations C of the pollutants corresponding to the different locations at the different time points based on the resolution for measuring pollutants. The pollutant sensor 132 may be, but not limited to, an optical finger navigation (OFN) sensor. For example, when the pollutant is carbon monoxide, the carbon monoxide will absorb the infrared rays with a wavelength of about 4700 nanometers generated by the OFN sensor. The carbon monoxide will reflect the infrared rays back to the OFN sensor. The difference between intensities of reflected and generated infrared rays is used to calculate the concentration of carbon monoxide. The air flow meter 133 measures the air flow rates V of the different locations in the physical environment 2. The processor 134 controls the pollutant sensor 132 to adjust the resolution for measuring pollutants based on the air flow rates V and transmits the concentrations C of the pollutants, the different time points, and the location related parameters including the RSRP, the SINRs, the packet RTT, the signal angles of arrival, the signal emission angles of departure, and the location of the wireless base station 11 to the cloud server 10 through the wireless communication module 130 and the wireless base station 11.

In other embodiments, the positioning pollutant-measuring device 13 may further include an inertial measurement unit (IMU) 135 electrically connected to the processor 134. The IMU 135 measures its displacement D, direction I, or acceleration A and transmits the displacement D, the direction I, or the acceleration A to the processor 134. The processor 134 transmits the displacement D, the direction I, or the acceleration A to the cloud server 10 through the wireless communication module 130 and the wireless base station 11. The cloud server 10 calculates the three-dimensional coordinates based on one of the displacement D, the direction I, and the acceleration A measured by the IMU 135. Besides, the positioning pollutant-measuring device 13 may further include an automatic angle adjusting machine 136 electrically connected to the processor 134 and combined with the pollutant sensor 132. The processor 134 controls the automatic angle adjusting machine 136 to adjust the sensing angle of the pollutant sensor 132, such that the pollutant sensor 132 accurately measures the concentration C of the pollutants. For example, the automatic angle adjusting machine 136 can horizontally or vertically rotate the pollutant sensor 132.

In some embodiments of the present invention, the positioning pollutant-measuring system 1 may further include a two-dimensional (2D) lidar 14 and an automatic height adjusting machine 15 lest obstacles affect the accuracy for measuring pollutants. The two-dimensional (2D) lidar 14 is electrically connected to the processor 134 and arranged on the automatic moving vehicle 12. The automatic height adjusting machine 15 is arranged between the automatic moving vehicle 12 and the positioning pollutant-measuring device 13 and electrically connected to the processor 134. The two-dimensional lidar 14 scans an obstacle around itself to obtain a relative distance RD between the two-dimensional lidar 14 and the obstacle. When the relative distance RD is less than a given distance, the processor 134 drives the automatic height adjusting machine 15 to increase a height where the positioning pollutant-measuring device 13 is located. When the relative distance RD is greater than or equal to the given distance, the processor 134 drives the automatic height adjusting machine 15 to decrease a height where the positioning pollutant-measuring device 13 is located.

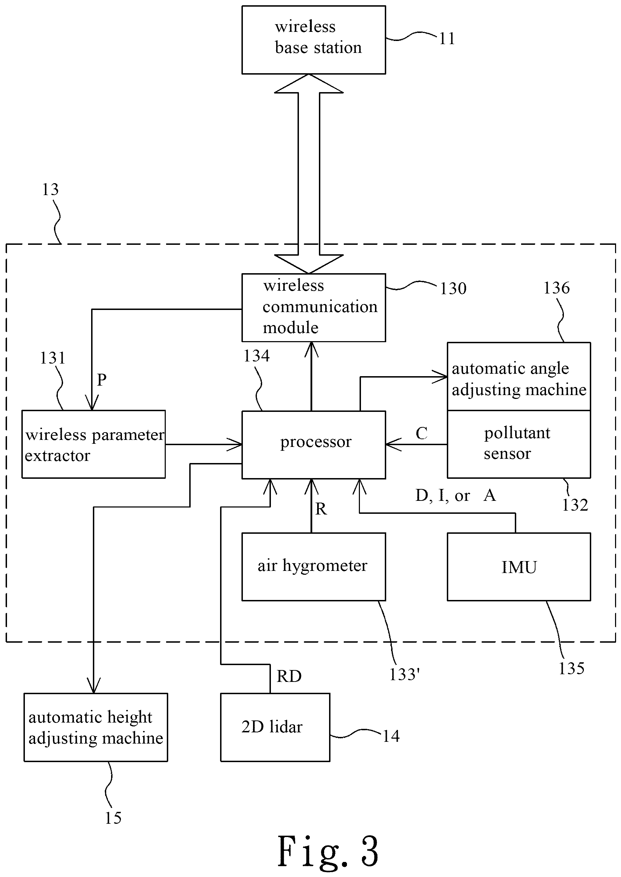

Fig.3 is a schematic diagram illustrating a positioning pollutant-measuring device according to anther embodiment of the present invention. Referring to Fig.3, the embodiment of Fig.3 is different from that of Fig.2 in that the air hygrometer 133' of Fig.3 replaces the air flow meter 133 of Fig.2. The air hygrometer 133', electrically connected to the processor 134, measures the air humidity R of the different locations in the physical environment 2. The processor 134 controls the pollutant sensor 132 to adjust the resolution for measuring pollutants based on the air humidity R. The other technical features of Fig.3 have been previously described so will not be reiterated.

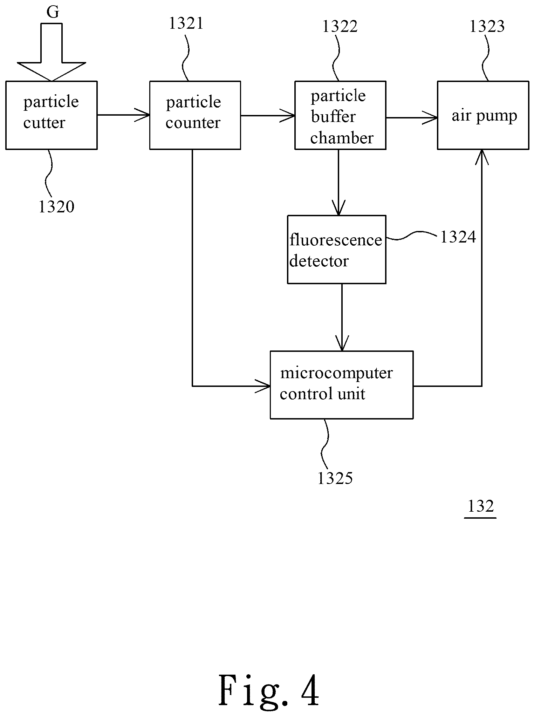

Fig.4 is a schematic diagram illustrating a pollutant sensor according to anther embodiment of the present invention. Please refer to Fig.3 and Fig.4. When the pollutant is a bioaerosol containing bacteria, mold, or viruses, the pollutant sensor 132 may include a particle cutter 1320, a particle counter 1321, a particle buffer chamber 1322, an air pump 1323, a fluorescence detector 1324, and a microcomputer control unit 1325. The particle cutter 1320, the particle counter 1321, the particle buffer chamber 1322, and the air pump 1323 are connected in sequence through gas pipelines. The particle buffer chamber 1322 is connected to the fluorescence detector 1324 through a gas pipeline. The microcomputer control unit 1325 is electrically connected to the processor 134, the particle counter 1321, and the air pump 1323. When the air pump 1323 starts to operate, the air pump 1323 absorbs a bioaerosol G and transmits it to the particle cutter 1320. The particle cutter 1320 can filter out particles, water droplets, oil droplets and smoke particles with a particle diameter greater than 10 microns (µm) in the sampled bioaerosol G to avoid interference caused by non-biological aerosols that can produce fluorescence, thereby reducing the error rate of the device. The particle counter 1321 may operate based on the light scattering principle. The particle counter 1321 is placed before the particle buffer chamber 1322 and after the particle cutter 1320 to determine the optical equivalent particle diameter and the concentration of the particles of the bioaerosol G to be measured. The fluorescence detector 1322 is used to detect the fluorescence intensity of the particle clusters of the bioaerosol G in the particle buffer chamber 1322. Specifically, the fluorescence detector 1322 includes an excitation light source and a photodetector. The photodetector is used to obtain the fluorescence intensity of the particle cluster of the bioaerosol G excited by the excitation light in the particle buffer chamber 1322, convert the fluorescence intensity into an electrical signal, and transmit the electrical signal to the microcomputer control unit 1325. The microcomputer control unit 1325 divides the fluorescence intensity by the atmospheric volume to obtain the concentration of the bioaerosol G and uses the concentration of the bioaerosol G as the concentration of pollutants. The pollutant sensor 132 can also refer to Chinese patent with application number CN201310009382.X.

According to the embodiments provided above, the positioning pollutant-measuring system positioning combines the different locations with the concentrations of the pollutants at different time points to implement environmental detection distribution in three-dimensional space.

## Claims

1. A positioning pollutant-measuring system (1) comprising:
a cloud server (10);
at least one wireless base station (11) electrically connected to the cloud server (10);
an automatic moving vehicle (12) configured to move in a physical environment (2) and pass through different locations in the physical environment (2); and
a positioning pollutant-measuring device (13) wirelessly connected to the at least one wireless base station (11) and arranged on the automatic moving vehicle (12), wherein the positioning pollutant-measuring device (13) is configured to obtain from the at least one wireless base station reference signal received power (RSRP), signal-to-interference-plus-noise ratios (SINRs), packet round-trip time (RTT), signal angles of arrival, signal emission angles of departure, and a location of the at least one wireless base station (11) corresponding to the different locations, the positioning pollutant-measuring device (13) is configured to measure air flow rates or air humidity of the different locations and adjust a resolution for measuring pollutants corresponding to the different locations based on the air flow rates or the air humidity, the positioning pollutant-measuring device (13) is configured to measure concentrations of the pollutants corresponding to the different locations at different time points based on the resolution and transmit the concentrations, the different time points, the RSRP, the SINRs, the packet RTT, the signal angles of arrival, the signal emission angles of departure, and the location of the at least one wireless base station (11) to the cloud server (10) through the at least one wireless base station (11), and the cloud server (10) is configured to calculate three-dimensional coordinates of the different locations based on the RSRP, the SINRs, the packet RTT, the signal angles of arrival, the signal emission angles of departure, and the location of the at least one wireless base station (11) and combine the three-dimensional coordinates with the corresponding time points and the corresponding concentrations.

2. The positioning pollutant-measuring system (1) according to claim 1, wherein the positioning pollutant-measuring device (13) comprises:
a wireless communication module (130) wirelessly connected to the at least one wireless base station (11);
a wireless parameter extractor (131) electrically connected to the wireless communication module (130) and configured to obtain the RSRP, the SINRs, the packet RTT, the signal angles of arrival, the signal emission angles of departure, and the location of the at least one wireless base station (11) from the at least one wireless base station (11) through the wireless communication module (130);
a pollutant sensor (132), having the resolution, configured to measure the concentrations of the pollutants corresponding to the different locations at the different time points based on the resolution;
an air flow meter (133) configured to measure the air flow rates of the different locations in the physical environment (2); and
a processor (134) electrically connected to the wireless parameter extractor (131), the wireless communication module (130), the pollutant sensor (132), and the air flow meter (133) and configured to control the pollutant sensor (132) to adjust the resolution based on the air flow rates and transmit the concentrations, the different time points, the RSRP, the SINRs, the packet RTT, the signal angles of arrival, the signal emission angles of departure, and the location of the at least one wireless base station (11) to the cloud server (10) through the wireless communication module (130) and the at least one wireless base station (11).

3. The positioning pollutant-measuring system (1) according to claim 2, wherein the pollutant sensor (132) is an optical finger navigation (OFN) sensor.

4. The positioning pollutant-measuring system (1) according to claim 2, wherein the positioning pollutant-measuring device (13) further comprises an inertial measurement unit (IMU) (135) electrically connected to the processor (134), the IMU (135) is configured to measure its displacement, direction, or acceleration and transmit the displacement, the direction, or the acceleration to the processor (134), the processor (134) is configured to transmit the displacement, the direction, or the acceleration to the cloud server (10) through the wireless communication module (130) and the at least one wireless base station (11), and the cloud server (10) is configured to calculate the three-dimensional coordinates based on one of the displacement, the direction, and the acceleration.

5. The positioning pollutant-measuring system (1) according to claim 2, wherein the positioning pollutant-measuring device (13) further comprises an automatic angle adjusting machine (136) electrically connected to the processor (134) and combined with the pollutant sensor (132) and the processor (134) is configured to control the automatic angle adjusting machine (136) to adjust a sensing angle of the pollutant sensor (132).

6. The positioning pollutant-measuring system (1) according to claim 2, further comprising:
a two-dimensional (2D) lidar (14) electrically connected to the processor (134) and arranged on the automatic moving vehicle (12), wherein the two-dimensional lidar (14) is configured to scan an obstacle around itself to obtain a relative distance between the two-dimensional lidar (14) and the obstacle; and
an automatic height adjusting machine (15) arranged between the automatic moving vehicle (12) and the positioning pollutant-measuring device (13) and electrically connected to the processor (134), wherein when the relative distance is less than a given distance, the processor (134) drives the automatic height adjusting machine (15) to increase a height where the positioning pollutant-measuring device (13) is located, and when the relative distance is greater than or equal to the given distance, the processor (134) drives the automatic height adjusting machine (15) to decrease a height where the positioning pollutant-measuring device (13) is located.

7. The positioning pollutant-measuring system (1) according to claim 1, wherein the positioning pollutant-measuring device (13) comprises:
a wireless communication module (130) wirelessly connected to the at least one wireless base station (11);
a wireless parameter extractor (131) electrically connected to the wireless communication module (130) and configured to obtain the RSRP, the SINRs, the packet RTT, the signal angles of arrival, the signal emission angles of departure, and the location of the at least one wireless base station (11) from the at least one wireless base station (11) through the wireless communication module (130);
a pollutant sensor (132), having the resolution, configured to measure the concentrations of the pollutants corresponding to the different locations at the different time points based on the resolution;
an air hygrometer (133') configured to measure the air humidity of the different locations in the physical environment (2); and
a processor (134) electrically connected to the wireless parameter extractor (131), the wireless communication module (130), the pollutant sensor (132), and the air hygrometer (133') and configured to control the pollutant sensor (132) to adjust the resolution based on the air humidity and transmit the concentrations, the different time points, the RSRP, the SINRs, the packet RTT, the signal angles of arrival, the signal emission angles of departure, and the location of the at least one wireless base station (11) to the cloud server (10) through the wireless communication module (130) and the at least one wireless base station (11).

8. The positioning pollutant-measuring system (1) according to claim 7, wherein the pollutant sensor (132) is an optical finger navigation (OFN) sensor.

9. The positioning pollutant-measuring system (1) according to claim 7, wherein the positioning pollutant-measuring device (13) further comprises an inertial measurement unit (IMU) (135) electrically connected to the processor (134), the IMU (135) is configured to measure its displacement, direction, or acceleration and transmit the displacement, the direction, or the acceleration to the processor (134), the processor (134) is configured to transmit the displacement, the direction, or the acceleration to the cloud server (10) through the wireless communication module (130) and the at least one wireless base station (11), and the cloud server (10) is configured to calculate the three-dimensional coordinates based on one of the displacement, the direction, and the acceleration.

10. The positioning pollutant-measuring system (1) according to claim 7, wherein the positioning pollutant-measuring device (13) further comprises an automatic angle adjusting machine (136) electrically connected to the processor (134) and combined with the pollutant sensor (132) and the processor (134) is configured to control the automatic angle adjusting machine (136) to adjust a sensing angle of the pollutant sensor (132).

11. The positioning pollutant-measuring system (1) according to claim 7, further comprising:
a two-dimensional lidar (2D) (14) electrically connected to the processor (134) and arranged on the automatic moving vehicle (12), wherein the two-dimensional lidar (14) is configured to scan an obstacle around itself to obtain a relative distance between the two-dimensional lidar (14) and the obstacle; and
an automatic height adjusting machine (15) arranged between the automatic moving vehicle (12) and the positioning pollutant-measuring device (13) and electrically connected to the processor (134), wherein when the relative distance is less than a given distance, the processor (134) drives the automatic height adjusting machine (15) to increase a height where the positioning pollutant-measuring device (13) is located, and when the relative distance is greater than or equal to the given distance, the processor (134) drives the automatic height adjusting machine (15) to decrease a height where the positioning pollutant-measuring device (13) is located.

12. The positioning pollutant-measuring system (1) according to claim 1, wherein the at least one wireless base station (11) is a 3G base station, a 4G base station, 5G base station, a Bluetooth (RTM) base station, or a WiFi base station.

13. The positioning pollutant-measuring system (1) according to claim 1, wherein the pollutants comprise bacteria, mold, viruses, flame, smoke, liquefied gas, carbon monoxide, carbon dioxide, ozone, alkane gases, benzene gases, ketone gases, natural gas, coal gas, gasoline, chlorine, ammonia, flammable gases, harmful volatile matter, or airborne matter.
